# EUROPEAN PATENT APPLICATION

(11) **EP 0 645 362 A1**
(43) Date of publication of application: **29.03.1995**
(21) Application number: 94114218.4
(22) Date of filing: 09.09.1994
(51) Int. Cl.: C07C 53/08, C07C 51/487, C07C 53/12, C07C 51/573

(54) **Process for producing highly purified acetic acid**

(30) Priority: 17.09.1993 JP 231340/93
(71) Applicant: DAICEL CHEMICAL INDUSTRIES, LTD., Sakai-shi, Osaka (JP)
(72) Inventor: Harano, Yoshiyuki, Himeji-shi, Hyogo (JP); Morimoto, Yoshiaki, Himeji-shi, Hyogo (JP)
(74) Representative: Grünecker, Kinkeldey, Stockmair & Schwanhäusser Anwaltssozietät

(57) **Abstract**

The present invention relates to a process for producing a highly purified acetic acid, which contains reducing substances in trace amounts and exhibits a satisfactory result in the permanganate reducing substance test, comprising the step of continuously reacting an aqueous solution of methanol and/or methyl acetate with carbon monoxide in a reactor, the step of continuously withdrawing a reaction fluid from the reactor and the step of evaporating the withdrawn reaction fluid in an evaporator to thereby obtain vaporous components containing acetic acid and distilling the vaporous components in a distillation column while withdrawing an overhead from the distillation column at its top, characterized in that the overhead is treated with ozone.

## Description

### Background of the Invention

### Technical Field

The present invention relates to an industrial process for producing a highly purified acetic acid. More particularly, the present invention is concerned with a process for continuously producing acetic acid having a lowered content of reducing impurities.

### Description of the Related Art

Acetic acid is a basic chemical which is used in large quantity in the petrochemical industry, organic chemical industry, pharmaceutical and agricultural chemical producing industry, polymer chemical industry and the like.

Various industrial processes are known for producing acetic acid. Among them, the process which comprises continuously reacting methanol with carbon monoxide to thereby produce acetic acid [see Herbert D. Grove, Hydrocarbon Process No. 11, 76 (1972)] is the most industrially advantageous one. According to this process, not only is the productivity of acetic acid high, but also the production of by-product impurities is reduced. Therefore, when acetic acid is produced by this process, acetic acid having a purity higher than those of the acetic acids produced by other processes can be obtained.

However, even if the above process is adopted, trace impurities are produced as the by-products. Accordingly, when a continuous operation is performed for a prolonged period of time even though acetic acid is produced according to the above process, by-product impurities contaminate acetic acid as the target product to thereby degrade the quality of the product. Therefore, even if the above process is adopted, large facilities are used and much energy is consumed for purification of acetic acid.

In recent years, a process with respect to the improvement of the catalyst for the production of acetic acid in which a catalyst stabilizer, such as an iodide salt, is added and another process in which methanol is reacted with carbon monoxide under such a condition that water content is lower than that of the prior art have been proposed as industrial processes for producing acetic acid at high productivity. It is stated that the amounts of by-products, such as carbon dioxide and propionic acid, are reduced when any of such processes is employed alone or such processes are employed in combination. However, as to some impurities produced as by-products in trace amounts, other than carbon dioxide and propionic acid, the amounts thereof are increased in accordance with an increase in the productivity of acetic acid. Therefore, when the productivity of acetic acid is enhanced by the above catalyst improvement or changes of reaction conditions, it is likely that the amounts of impurities other than carbon dioxide and propionic acid will increase to thereby degrade the quality of acetic acid.

The quality test called the permanganate reducing substance test (permanganate time) for checking the amounts of reducing impurities present in acetic acid even in trace amounts permits the detection of a slight increase in the amounts of trace impurities, the determination of which is difficult even by the analysis with modern high-performance instruments. This test has demonstrated that the quality of acetic acid is degraded by such reducing impurities. Examples of such impurities include aldehydes, especially unsaturated aldehydes. The aldehydes include a variety of compounds, and individual separation and removal thereof is not practical. In particular, crotonaldehyde, 2-ethylcrotonaldehyde and the like, which are condensates of acetaldehyde molecules through dehydration, have boiling points close to that of acetic acid, so that it is difficult to separate such trace impurities from acetic acid by distillation.

As processes for purifying acetic acid, one wherein crude acetic acid containing trace reducing impurities as mentioned above is treated with ozone [see Japanese Patent Publication-B No. 2052/1986 (January 22nd, 1986)] and another one wherein crude acetic acid containing trace reducing impurities as mentioned above is treated with an oxidizing agent have been disclosed. However, the treatment of crude acetic acid with ozone or an oxidizing agent has a drawback in that the concentrations and types of the impurities which can be treated are limited. For example, with respect to the treatment with ozone, there are drawbacks that (1) only unsaturated compounds can be treated with ozone, while saturated aldehydes are not decomposable by the treatment with ozone and that (2) the decomposition products resulting from the treatment of an unsaturated compound with ozone are saturated aldehydes, which also have reducing power, so that they are nothing but compounds becoming worse, i.e., shortening, the permanganate time. Therefore, after the treatment of crude acetic acid with ozone, a purification for removing the saturated aldehydes, such as treatment with active carbon, is further required [see European Patent No. 322215 (June 28th, 1989)].

### Disclosure of the Invention

### Summary of the Invention

The present inventors have studied means for removing reducing impurities contained in acetic acid in trace amounts. As a result, they have found that the reducing impurities can most effectively be removed by treating with ozone a fluid containing unsaturated compounds, which cause the permanganate time to be shortened, most concentrated therein in the acetic acid production process. Based on this finding, the present invention has been completed.

Thus, the present invention relates to a process for producing a highly purified acetic acid comprising the step of continuously reacting an aqueous solution of methanol and/or methyl acetate with carbon monoxide in a reactor, the step of continuously withdrawing a reaction fluid from the reactor and the step of evaporating the withdrawn reaction fluid in an evaporator to thereby obtain vaporous components containing acetic acid and distilling the vaporous components in a distillation column while withdrawing a distillate, i.e., an overhead, from the distillation column at its top, characterized in that the distillate, i.e., the overhead is treated with ozone.

After separating the distillate withdrawn from the top of the distillation column into an aqueous phase and an organic phase, the above treatment of the distillate with ozone may be conducted either only for the organic phase or only for the aqueous phase.

In the present invention, it is preferred that a rhodium compound and methyl iodide be used as a catalyst and a promoter, respectively.

That is, the present invention comprehends a process for producing a highly purified acetic acid, comprising continuously reacting an aqueous solution of methanol and/or methyl acetate with carbon monoxide with the use of a rhodium compound and methyl iodide as a catalyst and a promoter, respectively, to thereby obtain acetic acid, characterized in that a reaction fluid is continuously withdrawn from a reactor, vaporous components containing acetic acid as the desired product which is obtained by evaporation in an evaporator are distilled in a distillation column and a distillate which is withdrawn from the distillation column at its top is treated with ozone.

In this process, the above treatment of the distillate with ozone may be conducted either only for the organic phase forming a lower layer and mainly composed of methyl iodide or only for the aqueous phase forming an upper layer and mainly composed of water, after separating the distillate described above withdrawn from the top of the distillation column into the organic phase and the aqueous phase.

It has already been described that reducing substances in crude acetic acid shorten the permanganate time even if the content thereof is very small, and that when reducing substances are contained in the crude acetic acid in at least certain levels of concentrations, the treatment of the crude acetic acid with ozone does not lead to production of acetic acid exhibiting a satisfactory result in the permanganate reducing substance test. By contrast, a fluid in which unsaturated compounds are present in maximal concentrations in the acetic acid production process is treated with ozone in the process for producing acetic acid according to the present invention, so that the amount of reducing impurities in the crude acetic acid is effectively decreased by the above ozone treatment. Thus, when the process of the present invention is adopted, acetic acid of high quality can be produced only by conducting simple purification operation, such as distillation, after the ozone treatment.

### Brief Description of the Drawing

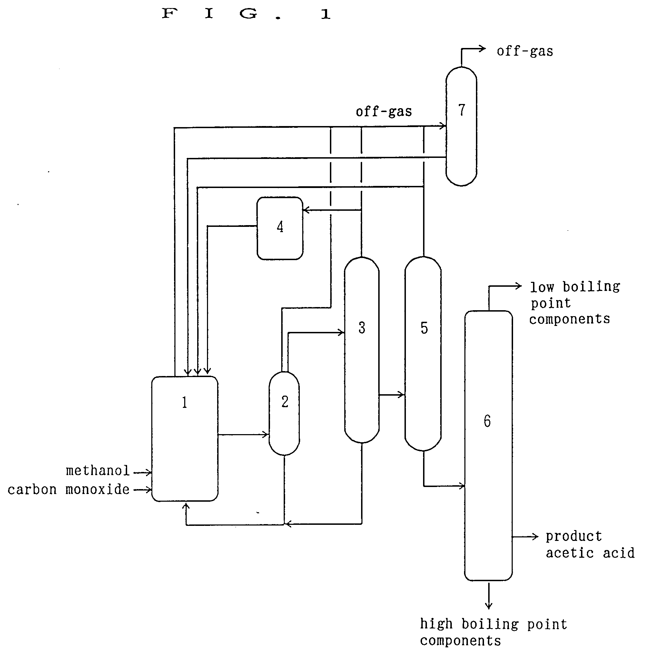

Fig. 1 is a flow diagram illustrating a process for continuously producing acetic acid.

In Fig. 1, numeral 1 designates a reactor (reaction vessel), numeral 2 an evaporator, numerals 3, 5 and 6 distillation columns, respectively, numeral 4 a liquid separator and numeral 7 an absorption system.

The scope and applicability of the present invention will be apparent from the following detailed description. However, it should be understood that the detailed description and specific examples, while indicating preferred embodiments of the invention, are given by way of illustration only, since various changes and modifications within the spirit and scope of the invention will become apparent to those skilled in the art from this detailed description.

### Detailed Description of the Invention

The process of the present invention can be practiced by adding one step (the step of ozone treatment) to the conventional processes, for example, the Monsanto process. First, referring to Fig. 1, the Monsanto process will be described.

The process for continuously producing acetic acid from methanol and carbon monoxide as starting compounds was disclosed by Monsanto Company.

Methanol and carbon monoxide as starting compounds are continuously fed into a reactor (or reaction vessel) (1), and continuously reacted each other at a predetermined temperature under a predetermined pressure. Generally, the reaction is effected under such conditions that the temperature is in the range of 150 to 250°C and the pressure is in the range of IS to 40 atm. Further, in the reactor (1), a catalyst, a promoter, a solvent, a reaction accelerator, etc., are generally put in addition to the above starting compounds.

The catalyst is not particularly limited as long as it is soluble in a reaction fluid (reaction mixture) comprising the above starting compounds and reaction products. Examples of the catalyst include rhodium compounds, palladium compounds, palladium complexes, molybdenum compounds, nickel compounds and the like. Further, compounds containing at least one compound(s) selected from the group consisting of cobalt, iridium, platinum, osmium and ruthenium can also be used as the catalyst. As the catalyst, only one compound or two or more compounds may be used. When a rhodium compound is used as the catalyst, those which are soluble in a reaction fluid and can form rhodium carbonyl species are generally used.

Examples of the rhodium compound usable in the present invention include
RhX₃ (wherein X represents Cl, Br or I),
RhX₃·3H₂O (wherein X represents Cl or Br),
Rh₂(CO)₁₆,
Rh₂(CO)₅,
Rh₂(CO)₄X₂ (wherein X represents Cl, Br or I),
Rh(CO)X[(C₆H₅)₃M]₂ (wherein X represents Cl, Br or I, and M represents P, As or Sb),
Rh(CO)₂X[(C₆H₅)₃M] (wherein X represents Cl, Br or I, and M represents P, As or Sb),
RhCl[(C₆H₅)₃P]₂(CH₃I)₂,
Rh(SnCl₃)[(C₆H₅)₃P]₃,
RhX[(C₆H₅)₃P]₃ (wherein X represents Cl, Br or I),
RhCl[(C₆H₅)₃P]₃H₂,
Rh[(C₆H₅)₃P₂(CO)I],
HRh(CO)[(C₆H₅)₃P]₃,
[(C₆H₅)P]₃Rh(CO)H,
[Rh(C₂H₄)₂Cl]₂,
K₄Rh₂X₂(SnX₃)₄ (wherein X represents Cl, Br or I),
Rh[(n-C₄H₉)₃P]₂(CO)X (wherein X represents Br or I),
[(n-C₄H₉)₄N][Rh(CO)₂X₂] (wherein X represents Cl, Br or I),
[(n-C₄H₉)₄As]₂[Rh₂(CO)₂X₄] (wherein X represents Br or I),
[(n-C₄H₉)₄P][Rh(CO)I₄],
and rhodium compounds described in Japanese Patent Publication-B No. 3334/1972 (Jan. 29th, 1972).

Examples of the palladium compound usable in the present invention include palladium chloride, palladium bromide, palladium iodide, palladium nitrate, palladium sulfate, palladium acetate, palladium propionate and palladium isobutyrate.

Examples of the palladium complex usable in the present invention include palladium acethylacetonate, sodium tetrachloropalladate, potassium tetrachloropalladate, potassium tetraiodepalladate, [Pd(CO)Cl₂]₂, [(n-C₄H₉)₄P]₂(PdCl₄), Pd[(C₆H₅)₃P]₂(CO)Br, Pd[(C₆H₅)₃P]₂I₂ and Pd[(n-C₄H₉)₃P]₂I₂.

An example of the molybdenum compound usable in the present invention includes hexacarbonylmolybdenum, and examples of the nickel compound include nickel iodide, nickel chloride and nickel acetate.

The catalysts described above may further include a ligand such as an amine, phosphorus, phosphine, arsine and stibine, optionally.

The catalyst is generally used to be the concentration thereof in the reaction fluid of from 100 to 10,000 ppm, preferably from 200 to 1,000 ppm.

The promoter is selected depending on the type of the catalyst employed. An example of the promoter includes methyl iodide. The promoter is used to be the amount thereof in the reaction fluid of from 5 to 20% by weight.

The reaction fluid generally comprises, in addition to the above components, 0.1 to 15% by weight of water as a solvent and acetic acid, mainly composed of the balance, as a reaction product and as a solvent. This reaction is continuously performed, so that methyl acetate, which is produced by reacting methanol as a starting compound with acetic acid, is present in the reaction fluid in a concentration range of 0.1 to 30% by weight.

Further, according to necessity, a reaction accelerator is used. Examples of the reaction accelerator include iodide compounds such as lithium iodide, sodium iodide, potassium iodide, rubidium iodide, cesium iodide, N-methylimidazole iodide and methyltributylphosphonium iodide, lithium compounds such as lithium acetate, Lewis acidic metal compounds such as aluminum compounds and chromium compounds and amide compounds such as N-dimethylacetamide and N-methylpyrolidone. When a rhodium compound is used as the catalyst, the reaction accelerator is generally added in an amount of 5 to 100, preferably 5 to 50 times by mole as much as the rhodium present in the rhodium compound. When a rhodium compound and an aluminum compound are respectively used as the catalyst and the reaction accelerator, an excellent effect is attained by adding an aluminum compound in an amount of about 10 to 20 times by mole as much as the rhodium.

When an aluminum compound is used as the reaction accelerator, a boron compound may be further added to stabilize the reaction accelerator. Examples of the boron compound include boric acid, metaboric acid and the like, which are each added in an amount of 1 to 10 times by mole as much as the aluminum present in the aluminum compound. Further, when an aluminum compound, a lithium compound or a Lewis acidic metal compound is used as the reaction accelerator, an iodide compound may be further added as the stabilizer of the catalyst.

The reaction fluid is continuously withdrawn from the reactor (1), and introduced into an evaporator (2) in which the pressure is maintained lower than the reaction pressure. In the evaporator (2), flash evaporation is conducted. The components evaporated in the evaporator are introduced into a distillation column (3), in which distillation is carried out. In the components evaporated in the evaporator, acetic acid is contained.

In the distillation column (3), low boiling point components and high boiling point components are separated and removed. Namely, a distillate of low boiling point components (overhead) is withdrawn from the top of the distillation column (3) and introduced into a liquid separator (4), while high boiling point components are withdrawn from the bottom of the distillation column and recycled to the reactor (1). The distillate introduced into the liquid separator (4) is separated into two layers. The upper layer is an aqueous phase containing acetic acid and the lower layer is an organic phase containing acetic acid and methyl acetate (when methyl iodide is used as the promoter, the organic phase is mainly composed of methyl iodide). These two phases are both recycled to the reactor (1).

A side cut fluid from the distillation column (3) containing acetic acid to be a product is fed into the following distillation column (5). The distillation column (5) is also called a dehydrating tower. In the distillation column (5), dehydration is performed to give crude acetic acid. The crude acetic acid thus obtained is withdrawn from the bottom of the column, and fed into the next distillation column (6). In the distillation column (6) as well, distillation is performed, and low boiling point components and high boiling point components are separated and removed therefrom. That is, in the distillation column (6), propionic acid as a high boiling point product and a trace of high boiling point impurities are withdrawn from the bottom of the column, and low boiling point components are withdrawn from the top of the column. The product acetic acid is obtained as a side cut from the distillation column (6). If desired, the distillation column (6) may be divided into a column for removing the high boiling point components and a column for removing the low boiling point components to thereby attain further purification of the acetic acid.

On the other hand, the offgas from the reactor (1) and the offgases (vented gases) from the evaporator (2) and from the distillation columns (3) and (5) contain not only useful components but also a trace of impurities, such as acetaldehyde. The impurities, such as acetaldehyde, are not separated from organic components contained in the offgases, such as methyl iodide, but both are recovered by an absorption system (7) and recycled to the reactor (1).

If a practical continuous production of acetic acid is conducted according to the above Monsanto process for a prolonged period of time, trace impurities, i.e., acetaldehyde and unsaturated aldehydes, such as crotonaldehyde and 2-ethylcrotonaldehyde, are accumulated in the reaction fluid. Further, although the reaction for synthesizing acetic acid is effected while keeping the water concentration of the reaction fluid low under highly productive conditions disclosed in recent years, it has been found that the production of unsaturated aldehydes among the above trace impurities as the by-products is increased. These unsaturated aldehydes are reducing compounds, which contaminate product acetic acid to thereby shorten the permanganate time. Therefore, removal of these compounds from the reaction process is important.

The unsaturated aldehydes, such as crotonaldehyde and 2-ethylcrotonaldelyde, are most concentrated in the overhead from the top of the distillation column (3), i.e., the upper and lower layers of the liquid separator (4). Therefore, if the overhead from the top of the distillation column (3) is not recycled to the reactor (1), a certain amount of unsaturated aldehydes can be removed from the reaction process. However, the overhead from the top of the distillation column (3) is recycled to the reactor (1) for reutilization, because it contains acetic acid and methyl acetate. Consequently, the unsaturated aldehydes are accumulated more and more in the reaction fluid.

The present inventors have studied effective removal of these compounds from the reaction process. As a result, it has been clarified that a highly purified acetic acid can be obtained by adding, to the conventional process for synthesyzing acetic acid, the step of treating with ozone the overhead from the top of the distillation column (3), i.e., the upper and lower layers of the liquid separator (4), having unsaturated aldehydes most concentrated therein to thereby decompose and remove the contained reducing compounds such as unsaturated aldehydes. More specifically, in the present invention, the upper layer fluid and/or the lower layer fluid of the liquid separator (4) is continuously contacted with ozone, an air containing ozone or a mixed gas of oxygen and nitrogen containing ozone to treat the fluid(s) with ozone. The upper layer fluid and/or the lower layer fluid of the liquid separator (4) after ozone treatment is separated from excess ozone, the air containing excess ozone or the mixed gas of oxygen and nitrogen containing excess ozone by passing through a gas liquid separator, and the separated fluid(s) is(are) recycled to the reactor (1). The amount of ozone to be used is at least equimolar to the total of the unsaturated compounds contained in the upper layer fluid and/or the lower layer fluid of the liquid separator (4).

In the acetic acid produced by the process of the present invention, reducing substances are contained in an extremely minute amount, so that the above acetic acid produced by the process of the present invention exhibits a satisfactory result in the permanganate reducing substance test. For obtaining acetic acid having an increased purity, however, the product acetic acid may be subjected to further purification operations, such as ozone treatment and distillation.

Further, the product acetic acid produced according to the present invention is not contaminated by aldol condensation products, which are formed by further reacting carbonyl impurities such as acetaldehyde and butylaldehyde and other impurities derived from acetaldehyde and butylaldehyde each other, and alkyl iodides such as hexyl iodide which are formed by reacting carbonyl impurities with iodide compounds as reaction accelerators.

### Examples

The present invention will be described in greater detail with reference to the following Examples, which should not be considered to limit the scope of the present invention.

### Example 1

One embodiment of the process for producing acetic acid will be described with the use of a continuous pilot plant as illustrated in Fig. 1.

The outline of the process employed is as follows.

Methanol and carbon monoxide as starting compounds are continuously fed to a reactor (1) containing 450 ppm of rhodium, 13% by weight of methyl iodide and 4.5% by weight of lithium iodide (concentrations in reaction fluid, respectively) fed, and continuously reacted each other at a temperature of 187 to 189°C under a pressure of 28 kg/cm². The reaction fluid is continuously withdrawn from the reactor (1), and introduced into an evaporator (2) in which the pressure is lower than the reaction pressure. Flash evaporation is conducted in the evaporator (2). Components evaporated in the evaporator (2) is introduced into a distillation column (3), in which distillation is performed. Low boiling point components are mainly separated in the distillation column (3). The distillate from the top of the distillation column is introduced into a liquid separator (4), and separated into two phases therein. The upper layer is an aqueous phase containing acetic acid, and the lower layer is an organic phase (methyl iodide phase) containing acetic acid and methyl acetate. Although these two phases are recycled to the reactor (1), the lower layer among them is recycled to the reactor (1) after treatment with ozone as will be described hereinbelow. High boiling point components from the distillation column (3) are also recycled to the reactor (1). A side cut fluid from the distillation column (3), containing acetic acid to be a product, is introduced into the following distillation column (5). Dehydration is conducted in the distillation column (5) to produce crude acetic acid. The crude acetic acid is withdrawn from the bottom of the distillation column (5), and introduced into the next distillation column (6), in which distillation is performed. In the distillation column (6), propionic acid as a high boiling point product and a trace of high boiling point impurities are withdrawn from the bottom of the column, and low boiling point impurities are withdrawn from the top of the column. A product acetic acid is obtained as a side cut fluid from the distillation column (6). On the other hand, organic components present in the offgas from the reactor (1) and in the offgases from the evaporator (2) and from the distillation columns (3) and (5) are recovered by an absorption system (7), and recycled to the reactor (1).

According to the above process, continuous reaction was carried out on a pilot plant having a capacity for producing 5 kg of acetic acid per hour. Under the above reaction conditions, an air containing 2000 ppm of ozone was blown at a rate of 50 ℓ per hour into a lower layer of the liquid separator (4) which was composed of 0.9% by weight of water, 3.5% by weight of acetic acid, 90.6% by weight of methyl iodide and 5.0% by weight of methyl acetate, containing traces of crotonaldehyde and 2-ethylcrotonaldehyde, to thereby effect the ozone treatment. The ozone-treated lower layer fluid was transferred to a gas liquid separator to thereby separate the fluid from the mixture of air and ozone gases, and the separated fluid was recycled to the reactor (1) by means of a pump. The permanganate time of the acetic acid obtained by the above process was 240 min or more. In the acetic acid, crotonaldehyde and 2-ethylcrotonaldehyde were contained only 0.1 ppm at the most in total.

### Example 2

Acetic acid was continuously produced by the same process and under the same reaction conditions as those of Example 1, except that the ozone treatment was conducted for an upper layer fluid of the liquid separator (4) in place of the lower layer fluid. Namely, the air containing 2000 ppm of ozone was blown at a rate of 50 ℓ per hour into an upper layer fluid of the liquid separator (4) which was composed of 48% by weight of water, 45% by weight of acetic acid and 7% by weight of methyl iodide and containing traces of crotonaldehyde and 2-ethylcrotonaldehyde. The ozone-treated upper layer fluid was transferred to a gas liquid separator to thereby separate the fluid from the mixture of air and ozone gases, and the separated fluid was recycled to the reactor (1) by means of a pump. The permanganate time of the acetic acid obtained by the above process was 240 min or more. In the acetic acid, crotonaldehyde and 2-ethylcrotonaldehyde were contained only 0.08 ppm at the most in total.

### Comparative Example

Acetic acid was continuously produced by the same process and under the same reaction conditions as those of Example 1, except that the lower layer fluid of the liquid separator (4) was not treated with ozone. The permanganate time of the acetic acid thus obtained was 100 min. Crotonaldehyde and 2-ethylcrotonaldehyde were contained in an amount of about 1 ppm in total.

## Claims

1. A process for producing a highly purified acetic acid comprising the step of continuously reacting an aqueous solution of methanol and/or methyl acetate with carbon monoxide in a reactor, the step of continuously withdrawing a reaction fluid from the reactor and the step of evaporating the withdrawn reaction fluid in an evaporator to thereby obtain vaporous components containing acetic acid and distilling the vaporous components in a distillation column while withdrawing an overhead from the distillation column at its top, characterized in that the overhead is treated with ozone.

2. The process for producing a highly purified acetic acid according to claim 1, wherein after separating the overhead which is withdrawn from the top of the distillation column into an aqueous phase and an organic phase, the organic phase is treated with ozone.

3. The process for producing a highly purified acetic acid according to claim 1, wherein after separating the overhead which is withdrawn from the top of the distillation column into an aqueous phase and an organic phase, the water phase is treated with ozone.

4. The process for producing a highly purified acetic acid according to claim 1, wherein a rhodium compound and methyl iodide are used as a catalyst and a promoter, respectively.

5. The process for producing a highly purified acetic acid according to claim 4, wherein after separating the overhead which is withdrawn from the top of the distillation column into an aqueous phase and an organic phase, the organic phase which forms a lower layer and is mainly composed of methyl iodide, is treated with ozone.

6. The process for producing a highly purified acetic acid according to claim 4, wherein after separating the overhead which is withdrawn from the top of the distillation column into an aqueous phase and an organic phase, the aqueous phase which forms an upper layer and is mainly composed of water is treated with ozone.
